# EUROPEAN PATENT APPLICATION

(11) **EP 4 491 712 A1**
(43) Date of publication of application: **15.01.2025**
(21) Application number: 24164881.5
(22) Date of filing: 20.03.2024
(51) Int. Cl.: C12N 5/077, G01N 33/569

(54) **METHOD FOR DETECTING CONTAMINANT CELLS IN A STEM CELL DERIVED CELL POPULATION**

(71) Applicant: Novo Nordisk A/S, 2880 Bagsvaerd (DK)
(72) Inventor: The designation of the inventor has not yet been filed

(57) **Abstract**

The present invention relates generally to stem cells, particularly to method for detecting contaminant stromal like cells in a population of non-stromal cells differentiated from stem cells. The present invention also relates to a culture medium for increasing the proportion of stromal like cells in a population of non-stromal cells differentiated from human pluripotent stem cells.

## Description

### TECHNICAL FIELD

The present invention relates to the field of stem cells. Particularly, it relates to a method for detecting specific type of contaminant cells in a population of cells differentiated from stem cells.

### BACKGROUND

Human Pluripotent Stem Cells (hPSC) can replicate indefinitely while retaining the capacity to differentiate into all cell types of the human body, for example pancreatic endocrine cells (PEC), cardiomyocytes etc. Step wise *in vitro* differentiation of hPSC is carried out to obtain an inexhaustible source of any desired cell type to be explored as transplantable stem cell therapy products for treatment of various diseases.

However, the major impediment in using the desired cells as a cell therapy product is the potential safety risk due to contamination with unwanted cells. One type of unwanted cells or contaminant is an off-target cell population(s) of stromal like cells. Contaminant stromal like cells can arise during several stages of the differentiation of hPSC into desired cells. Accordingly, highly sensitive and specific methods to detect such contaminant cells is a critical safety requirement of hPSC-derived cell therapy products for treatment of various diseases.

However, there remains a need for efficient techniques for identifying contaminant stromal like cells in a population of cells differentiated from hPSCs.

### SUMMARY

The present invention provides an *in vitro* method for detecting or identifying contaminant cells in a population of cells differentiated from stem cells.

The present invention relates to a stromal like cell assay.

In one aspect, the present invention provides a method for identifying contaminant stromal like cells in a population of non-stromal cells differentiated from human pluripotent stem cells comprising the steps of:
(i) selectively expanding the stromal like cells in the population of cells in a culture medium comprising one or more growth factors and/or supplements, and
(ii) detecting the contaminant stromal like cells.

In one aspect, detecting the contaminant stromal like cells is by way of identifying one or more markers of the stromal like cells selected from a group consisting of COL1A1, COL1A2, COL3A1, COL6A1, COL6A2, FN1, THY1, VIM, NES, CDH2, PDGFRA, PDGFRB, MEIS1, SIX1, PAX2, PAX8, PRRX1, PCOLCE, VCAN, DDR1, DDR2, SPARC and CDH11.

In another aspect, the present invention provides a culture medium for selectively expanding the proportion of stromal like cells within a population of cells comprising non-stromal cells differentiated from human pluripotent stem cells.

In further aspect, the present invention provides a cell population comprising non-stromal cells differentiated from human pluripotent stem cells and contaminant stromal like cells, wherein less than 1% of the cells are contaminant stromal like cells, for use as a medicament.

### BRIEF DESCRIPTION OF DRAWINGS

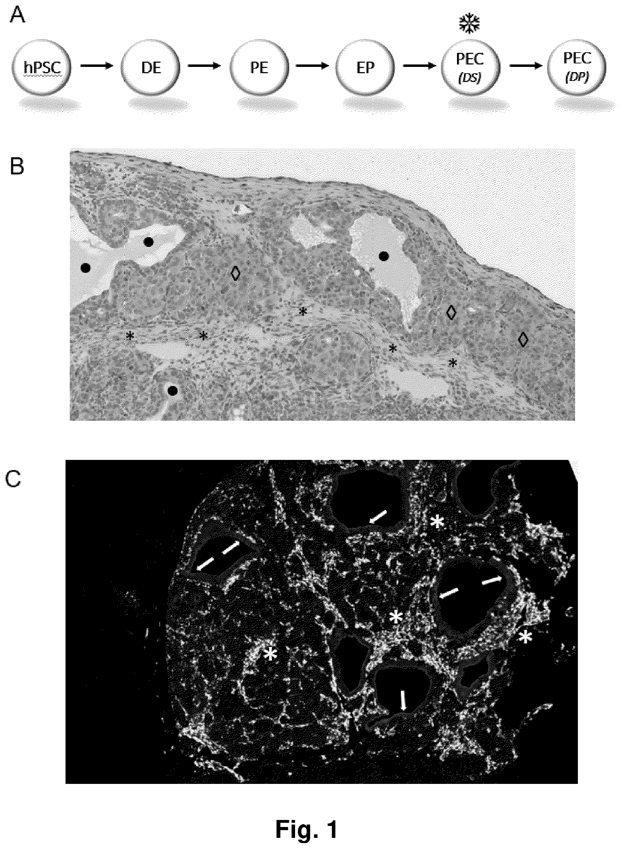

Fig. 1A shows a schematic overview of differentiation of hPSCs to pancreatic endocrine cells (PEC). hPSCs can be differentiated by several known protocols. In one of the protocols hPSCs are first differentiated to definitive endoderm (DE), DE is differentiated to pancreatic endoderm (PE) and PE is differentiated to endocrine progenitors (EP) and finally, endocrine progenitors are differentiated towards pancreatic endocrine cells. PEC (DS): Pancreatic endocrine cells are dissociated and cryopreserved and termed PEC drug substance (DS) at this stage. PEC (DP): Cryopreserved pancreatic endocrine cells (DS) are thawed and subsequently reaggregated in culture medium for several days to form PEC clusters termed PEC drug product (DP). PEC (DP) is used for *in vivo* studies.
Fig. 1B shows H&E staining of kidney capsule graft. PEC (DP) clusters were transplanted under the kidney capsule of immunocompromised mice. PEC (DP) clusters were matured for ~5 weeks in vivo and grafted kidneys were subsequently processed for histological assessment of grafted cells. Image shows hematoxylin and eosin (H&E) staining of grafted cells. 0 marks pancreatic endocrine clusters. • marks cyst/duct-like structures demarcated by an epithelial cell layer. * marks fibrotic/stromal-like tissue.
Fig. 1C shows immunostaining of kidney capsule graft on adjacent section of graft from the same animal shown in B). Grafted cells were assessed for expression of the fibrotic/stromal marker Vimentin (VIM) and epithelial cyst/duct marker cytokeratin 19 (CK-19). White * highlights adjacent areas positive for VIM. White arrows → highlight areas that are CK-19 positive.

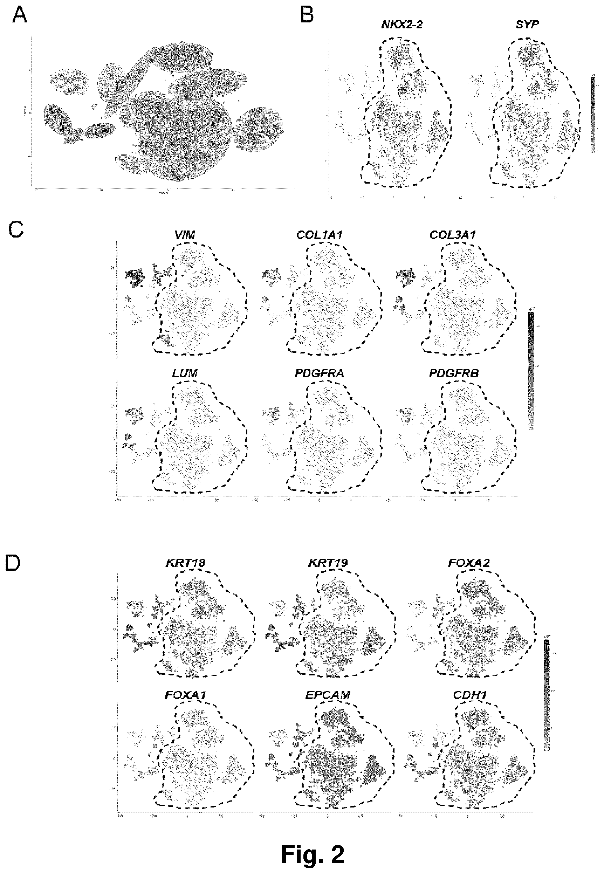

Fig. 2A shows t-distributed stochastic neighbor embedding (t-SNE) visualization of single-cell RNA sequencing (scRNA_seq) data set generated from multiple PEC (DP) samples. Cluster analysis identified 13 distinct clusters based on gene expression patterns.
Fig. 2B shows expression levels of the pan-endocrine cells genes *NKX2-2* and *SYP* in single cells. Endocrine cells are located in cluster 1, 9-13 shown in A) and demarcated with dashed lines. In B) Cells outside the dashed lines (cluster 2-8 in A) are non-endocrine cells.
Fig. 2C shows expression levels of canonical stromal like cell genes VIM, COL1A1, COL3A1, LUM, PDGFRA, PDGFRB in single cell RNA data set. Cells primarily located in cluster 3 are co-expressing these markers. Cells outside the dashed lines are non-endocrine cells.
Fig. 2D shows expression levels of canonical ductal, endodermal and epithelial cell genes KRT18, KRT19, FOXA2, FOXA1, EPCAM, CDH1 in single cell RNA data set. Cells primarily located in cluster 2, 4, 6-8 are co-expressing these markers. Cells outside the dashed lines are non-endocrine cells.

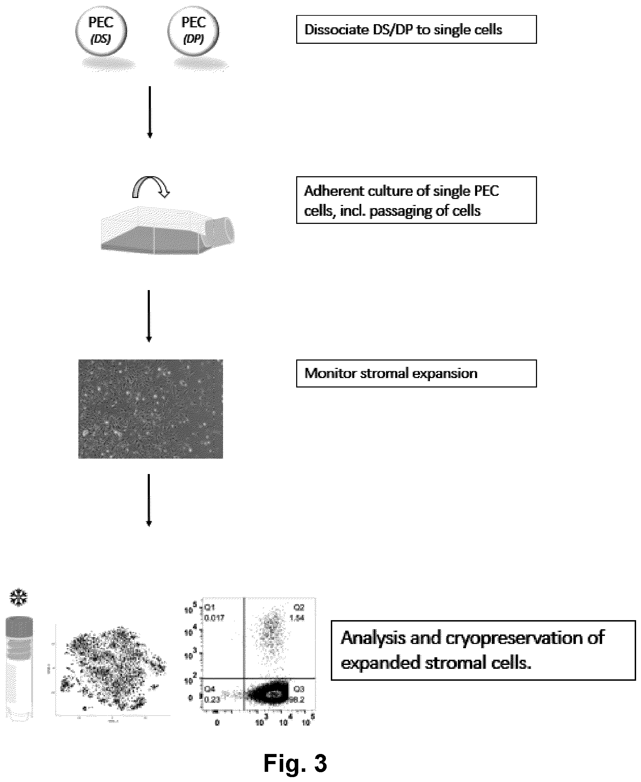

Fig. 3 shows schematic overview of stromal like cell assay. Single cells from DS and/or DP are obtained by enzymatic dissociation of PEC clusters to a single cell suspension. Single cells are subsequently cultured on tissue culture plasticware in a medium promoting selective expansion of stromal like cells. Passaging of cells is possible and allows for enrichment and further expansion of stromal like cells. Expansion of stromal like cells is easily observed and monitored by bright field microscopy. Stromal like cells can be harvested for subsequent analysis as well as cryopreservation. Analysis includes various gene and protein expression assays as well as functional assays.

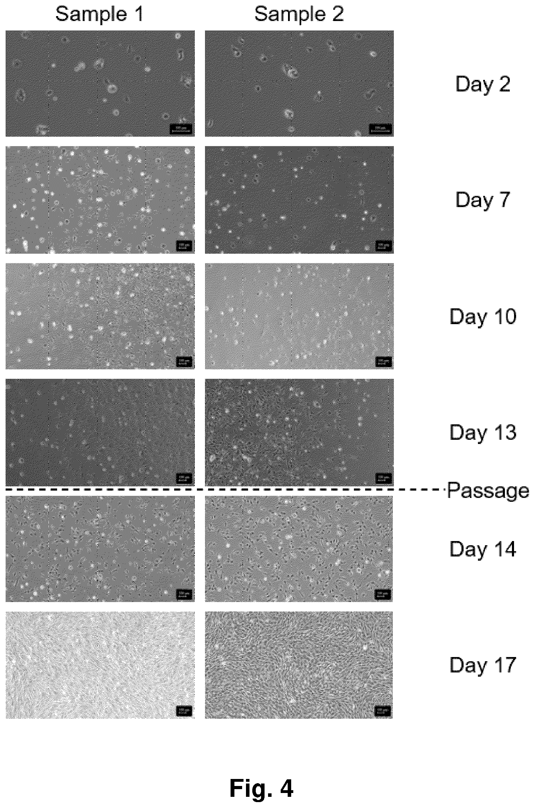

Fig. 4 shows examples of stromal like cell expansion over time. Bright field microscopy images of two distinct samples of PEC DP clusters dissociated to single cells and subsequently seeded and cultured in stromal like cell assay. Days indicate number of days in culture since initial seeding of PEC DP. Dotted line indicates passaging of cells on day 13.

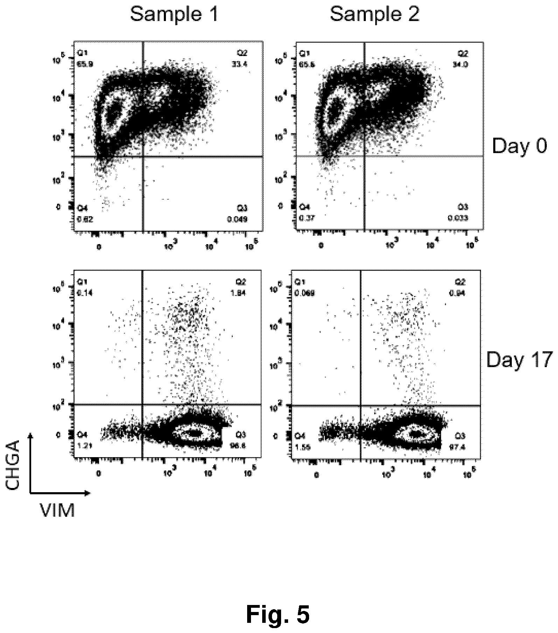

Fig. 5 shows flow cytometry analysis of PEC DP at the time of seeding cells in stromal like cell assay (Day 0) and after 17 days of culture in stromal like cell assay, including one passage of cells (day 17). Cells were analysed for protein expression of Chromogranin A (CHGA) and Vimentin (VIM). CHGA is a marker of endocrine cells whereas Vimentin is applied as a marker for stromal like cells. At day 0 the majority (>99%) of the cells are endocrine (CHGA+). However, following expansion of stromal like cells for 17 days, the proportion of stromal like cells expressing Vimentin increased (>95%) as compared to endocrine cells expressing CHGA (<2%).

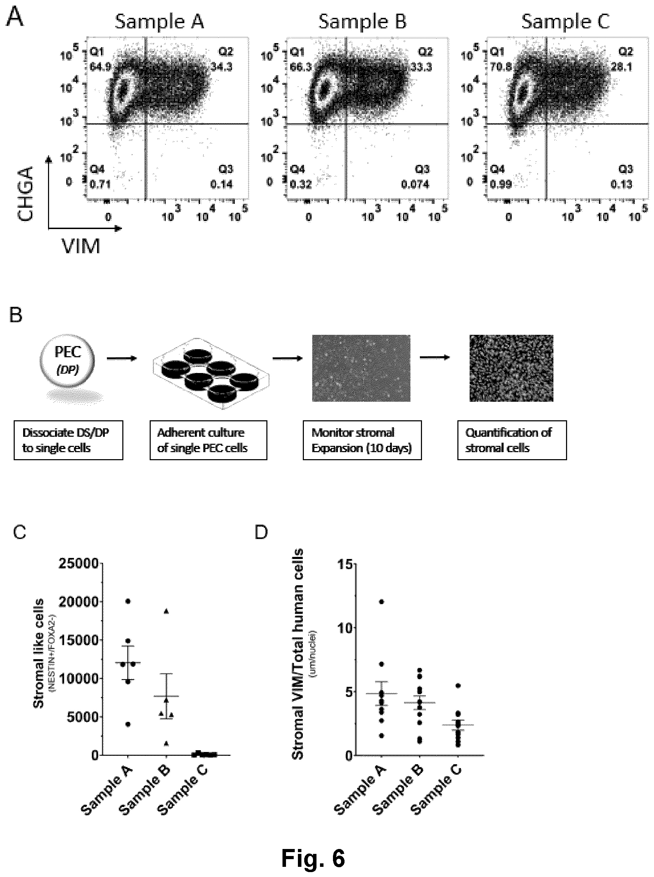

Fig.6A shows flow cytometry analysis of three different PEC drug products (sample A, B and C). Cells were analysed for protein expression of Chromogranin A (CHGA) and Vimentin (VIM). CHGA is a marker of endocrine cells whereas Vimentin is applied as a marker for stromal like cells. The flow cytometry analysis is not sensitive enough to detect differences in stromal like cell numbers between the three PEC drug products.
Fig.6B shows an outline of stromal like cell assay. The three PEC drug products shown in A) were seeded into 6-well tissue culture plates (6 wells pr. sample) and cultured for 10 days. At the end of the culture period, cells were fixed and subsequently stained for a marker of stromal like cells (NESTIN) and a marker of endocrine cells (FOXA2). The number of stromal like cells in each well of the tissue culture plates were quantified using a cell imaging reader platform.
Fig. 6C shows quantification of stromal like cells expanded from the three PEC drug product samples shown in A). Graph shows the total number of stromal like cells detected in each well (six wells pr. sample, symbols represent individual wells). Mean ± standard error of the mean is also shown for each sample. The stromal like cell assay allows to detect differences in stromal like cell expansion from the three samples, ranking sample A > sample B > sample C with regards to the amount of contaminating stromal like cells.
Fig. 6D shows *in vivo* outcome of transplantation of PEC drug products. The three PEC drug products shown in A) were transplanted under the kidney capsule of immunocompromised mice. 12 weeks following transplantation, kidneys were harvested and histologically assessed for contaminating stromal like cells. Vimentin (VIM) was used as a marker to determine the volume of stromal like cells (µM) and normalized to the total amount of human cells (nuclei). PEC drug products are ranked sample A > sample B > sample C from most to least amount of contaminating stromal like cells in the kidney capsule grafts. Thus, the stromal like cell assay quantification correctly predicted the *in vivo* outcome of contaminating stromal like cells from the three PEC drug products.

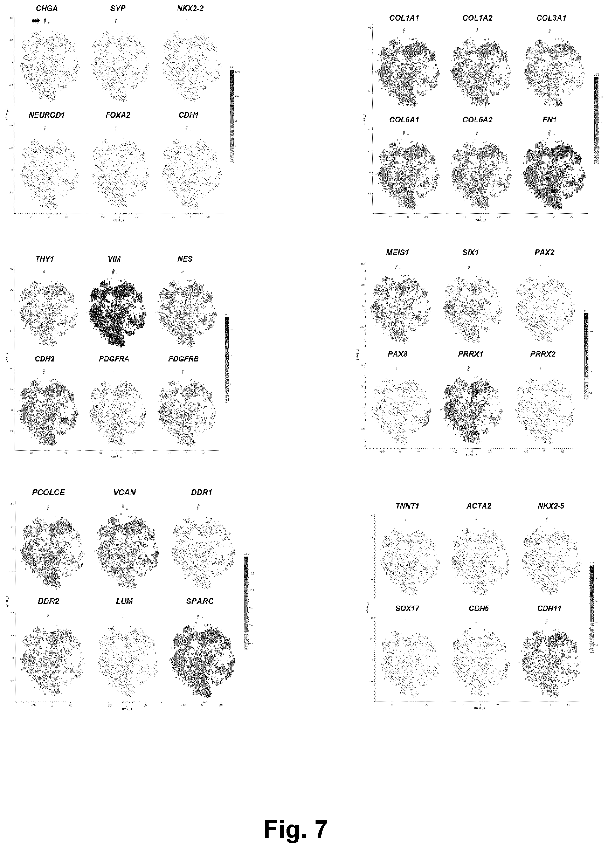

Fig. 7 shows stromal like cells expanded from two distinct PEC drug substances were subjected to single cell RNA sequencing. Plots in upper left corner evaluates six pancreatic endocrine and epithelial markers in expanded stromal like cells and highlights a small fraction of endocrine cells still present after expansion (arrow). Remaining plots evaluates genes for stromal like cells and other lineages, including kidney, endothelial and cardiomyocytes.

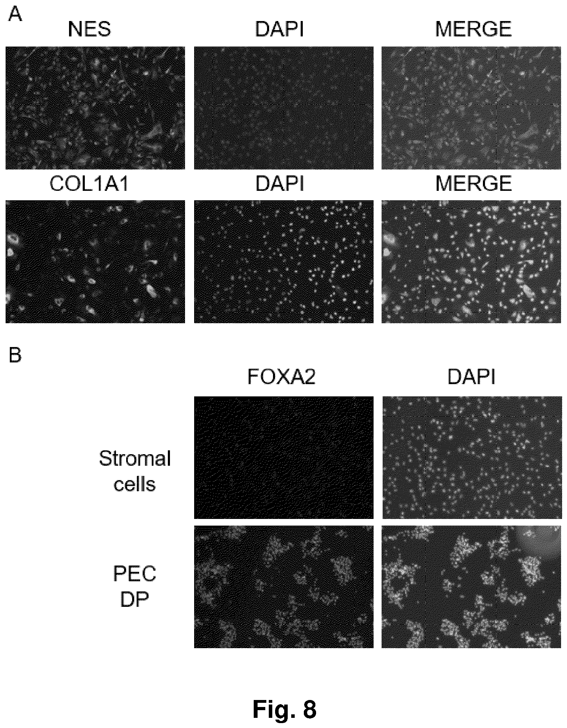

Fig. 8A shows immunofluorescence microscopy evaluation of Nestin (NES) and COL1A1 expression in expanded stromal like cells. DAPI labels all nuclei.
Fig. 8B shows immunofluorescence microscopy evaluation of FOXA2 in expanded stromal like cells as well as PEC DP cells. Only PEC DP stains positive for FOXA2. DAPI labels all nuclei.

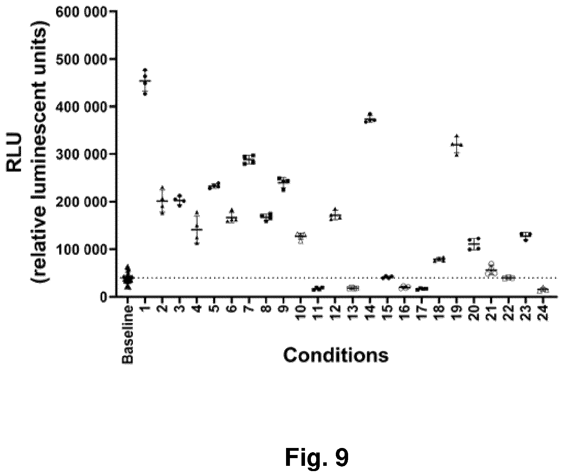

Fig. 9 shows CellTiter-Glo luminescent cell viability assay results of the combinatorial medium, supplement, matrix and growth factor screen corresponding to table 2 in example 4. Dotted line marks mean day 0 (CellTiter-Glo analysis 2h post seeding) value, which represent the luminescent value of the cells seeded at day 0. Values above the dotted line indicates cell growth across the four days of the assay.

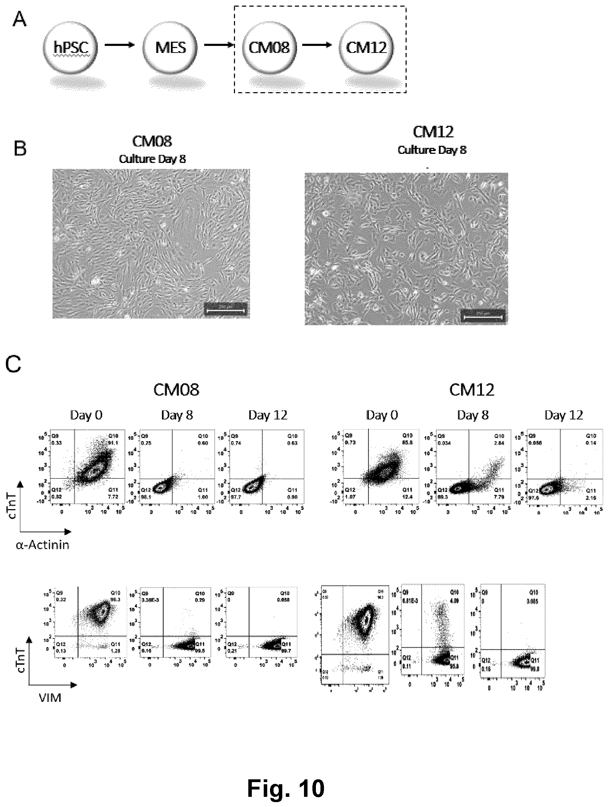

Fig. 10A shows Schematic overview of the hPSC-Cardiomyocyte differentiation protocol. CM08 = Cardiomyocytes, day 8 of the differentiation. CM12 = Cardiomyocytes, day 12 of the differentiation.
Fig. 10B shows representative images of stromal-like cells at day 8 following seeding of CM08 and CM12 cells in stromal like cell assay.
Fig. 10C shows flow cytometry analysis of the cardiomyocyte markers cTnT/α-Actinin (top panels) and cTnT and the stromal like cell marker VIM (bottom panel). CM08 and CM12 cardiomyocytes were seeded into the stromal assay (day 0) and cultured for 12 days. Flow cytometry samples were taken on day 0 (day of seeding cells), day 8 and day 12 of culture in the stromal like cell assay.

### DESCRIPTION

Several drug products in form of fully differentiated cells derived from hPSCs are currently being explored as a cellular therapy. Contaminant cells can arise during several stages of hPSC differentiation and even a small amount of contaminant cells in the final drug product can be a potential safety concern. Conventional methods such as flow cytometry, quantitative PCR, digital droplet PCR and fluorescence microscopy can be applied to measure a marker(s) specific to contaminant cells directly in the drug product. Thus, to detect contaminant cells such assays rely on the contaminant cells being present in the drug product at levels above the lower limit of detection and lower limit of quantification (LLOD and LLOQ) for the specific assays. However, assays or detection techniques mentioned above often do not have the required sensitivity and specificity. To ensure that a hPSC-derived drug product is safe it is important to demonstrate that contaminant cells are either absent or minimally present in the drug product. Thus, there has been a need to develop highly sensitive assays that can reliably detect contaminant cells in a drug product.

The present invention relates to a cell culture medium that allows for the selective expansion of stromal-like cells present in hPSC-derived non-stromal drug products. Since the expansion is specific to the stromal-like cells which is an off-target population, selectively the stromal like cells will grow but not the non-stromal cells. Hence, the proportion between stromal-like cells and drug product will increase. Thus, the selective expansion will allow for an increased sensitivity for the stromal-like cells in hPSC-derived drug products. The expanded stromal-like cells can subsequently be detected, characterized, and quantified using various conventional detection methods.

The combination of the cell culture system for selective expansion of stromal like cells and conventional methods for detecting stromal-like cells can reliably identify and quantify stromal-like cells with a significantly higher sensitivity and specificity than the conventional methods alone.

Unless otherwise stated, all technical and scientific terms used herein have the same meanings as commonly understood by one of ordinary skill in the art to which this invention belongs. The practice of the present invention employs, unless otherwise indicated, conventional methods of chemistry, biochemistry, biophysics, molecular biology, cell biology, genetics, immunology, and pharmacology, known to those skilled in the art.

It is noted that all headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed.

### DEFINITIONS

### General definitions

As used herein, "a" or "an" or "the" can mean one or more than one. Unless otherwise indicated in the specification, terms presented in singular form also include the plural situation.

As used herein, "and/or" refers to and encompasses any and all possible combinations of one or more of the associated listed items, as well as the lack of combinations when interpreted in the alternative ("or"). Moreover, the present invention also contemplates that in some embodiments of the invention, any feature or combination of features set forth herein can be excluded or omitted.

### Stem cells

As used herein, the term "stem cell" refers to an undifferentiated cell having proliferative capacity (particularly self-renewal competence) but maintaining differentiation potency. The term "stem cell" includes categories such as pluripotent stem cell, multipotent stem cell, and the like according to their differentiation potentiality.

As used herein, the term "pluripotent stem cell" (PSC)" refers to a stem cell capable of being cultured *in vitro* and having a potency to differentiate into any cell lineage belonging to the three germ layers (ectoderm, mesoderm, endoderm) and/or extraembryonic tissue (pluripotency). Examples of the pluripotent stem cell (PSC) include embryonic stem cell (ESC), EG cell (embryonic germ cell), induced pluripotent stem cell (iPSC) and the like.

In one embodiment, the cells of the method of the present invention are pluripotent stem cells.

As used herein, "human pluriopotent stem cell" (hPSC) refers to human pluripotent stem cells that can be derived from any source and that are capable, under appropriate conditions, of producing human progeny of different cell types that are derivatives of all the three germ layers (endoderm, mesoderm, and ectoderm).

In one embodiment, the cells of the method of the present invention are human pluripotent stem cells.

As used herein, the term "induced pluripotent stem cell" (also known as iPS cells or iPSCs) means a type of pluripotent stem cell that can be generated directly from cells that are not PSCs and have a nucleus. By the introduction of products of specific sets of pluripotency associated genes non-pluripotent cells can be converted into pluripotent stem cells.

In one embodiment, the cells of the method of the present invention are induced pluripotent stem cells.

As used herein, the term "embryonic stem cell" means a pluripotent stem cell derived from parthenotes as described in e.g. WO 2003/046141, the contents of which are incorporated by reference in their entirety. Additionally, embryonic stem cells can be produced from a single blastomere or by culturing an inner cell mass obtained without the destruction of the embryo. Embryonic stem cells are available from given organizations and are also commercially available. Preferably, the methods and products of the present invention are based on human PSCs, i.e. stem cells derived from either human induced pluripotent stem cells or human embryonic stem cells, including parthenotes.

As used herein, the term "multipotent stem cell" means a stem cell having a potency to differentiate into plural types of tissues or cells, though not all kinds and is typically restricted to one germ layer.

As used herein, the term "unipotent stem cell" means a stem cell having a potency to differentiate into only one specific tissue or cell.

As used herein, the term "non-native" means that the cells although derived from pluripotent stem cells, which may have human origin, is an artificial construct, that does not exist in nature. In general, it is an object within the field of stem cell therapy to provide cells, which resemble the cells of the human body as much as possible. However, it may never become possible to mimic the development which the pluripotent stem cells undergo during the embryonic and fetal stage to such an extent that the mature cells are indistinguishable from native cells of the human body. Inherently, in an embodiment of the present invention, the cells are artificial.

As used herein, the term "artificial" in reference to cells may comprise material naturally occurring in nature but modified to a construct not naturally occurring. This includes human stem cells, which are differentiated into non-naturally occurring cells mimicking the cells of the human body.

As used herein, the term "undifferentiated" pluripotent stem cell or human pluripotent stem cell or induced pluripotent stem cell means that such cell has not differentiated into another cell type.

As used herein, the term "stromal like cells" means cells that originate from the mesodermal germ layer. Neural crest and germ layers other than mesoderm can also give rise to certain stromal-like cell types. The stromal-like cells resemble cell types of human connective tissue. The most common stromal-like cell type is fibroblasts that produces extracellular matrix and collagens. Other stromal-like cells include but are not limited to pericytes, mesenchymal stromal cells, adipocyte stromal cells, endothelial cells, smooth muscle cells.

In one embodiment, stromal like cells are fibroblasts.

As used herein, the stromal-like cells are characterized by expression of one or more markers including but not limited to COL1A1, COL1A2, COL3A1, COL6A1, COL6A2, FN1, THY1, VIM, NES, CDH2, PDGFRA, PDGFRB, MEIS1, SIX1, PAX2, PAX8, PRRX1, PCOLCE, VCAN, DDR1, DDR2, SPARC and CDH11.

In one embodiment, stromal-like cells are NESTIN⁺ cells.

In another embodiment, stromal-like cells are CHGA⁻/NESTIN⁺ cells.

In one embodiment, stromal-like cells are cTNT⁻/NESTIN⁺ cells.

In another embodiment, stromal-like cells are CHGA⁻/cTNT⁻/NESTIN⁺ cells.

As used herein, the term "contaminant" cells mean any cells or cell type that are undesirable or unwanted in a cell population.

In one embodiment, contaminant cells are non-endocrine cells.

In one embodiment, the contaminant cells are stromal-like cells.

In another embodiment, the contaminant cells are fibroblasts.

As used herein, the term "off-target cell population" refers to a cell population of undesired cells that may be of same type or a mixture of different type of cells or cell lineages.

In one embodiment, stromal cells is an off-target cell population.

As used herein, the term "drug substance" refers to an active ingredient that is intended to furnish biological activity or other direct effect in the diagnosis, cure, mitigation, treatment or prevention of disease or to affect the structure or any function of the human body.

In one embodiment, the drug substance is non-stromal cells.

In one embodiment, the drug substance is PEC.

In one embodiment, the drug substance is cardiomyocytes.

In one embodiment, the drug substance is dopaminergic progenitors.

In one embodiment, the drug substance is retinal pigmented epithelial cells.

As used herein, the term "drug product" refers to finished dosage form that contains drug Substance, generally in association with one or more other ingredients (e.g. excipients).

In one embodiment, the drug product is non-stromal cells.

In one embodiment, the drug substance is PEC.

In one embodiment, the drug product is cardiomyocytes.

In one embodiment, the drug product is dopaminergic progenitors.

In one embodiment, the drug product is retinal pigmented epithelial cells.

As used herein, the term "non-stromal cells" refers to cells that are not stromal-like cells.

In one embodiment, non-stromal cells are cells differentiated from human pluripotent stem cells.

In one embodiment, non-stromal cells are pancreatic endocrine cells.

In one embodiment, non-stromal cells are cardiomyocytes.

In one embodiment, non-stromal cells are dopaminergic progenitors.

In one embodiment, non-stromal cells are retinal pigmented epithelial cells.

### Expression of markers

As used herein, the term "expressing or expression" in relation to a gene or protein refers to the presence of an RNA molecule, which can be detected using assays such as reverse transcription quantitative polymerase chain reaction (RT-qPCR), RNA sequencing and the like, and/or a protein, which can be detected for example using antibody-based assays such as flow cytometry, immunocytochemistry/immunofluorescence, and the like.

Depending on the sensitivity and specificity of the assay, a gene or protein may be considered expressed when a minimum of one molecule is detected such as in RNA sequencing, or the limit of detection above background/noise levels may be defined in relation to control samples such as in flow cytometry. A person skilled in the art will readily appreciate that a cell can be defined by the positive or negative expression of a marker, i e. the properties and state of a cell may equally be correlated based on the expression of a certain marker as well as the lack thereof. When referring to specific markers the presence or lack of expression may be denoted with + (plus) or - (minus) signs, respectively.

As used herein, the term "expression level" refers to the degree of gene expression and/or gene product activity in a cell. Expression level can be determined in arbitrary absolute units or normalized units (relative to known expression levels of a control reference).

As used herein, the term "marker" refers to a naturally occurring identifiable expression made by a cell which can be correlated with certain properties of the cell and serves to identify, predict or characterise a cell or cell population. A marker may be referred to by gene. A marker may be in the form of mRNA or protein for e.g., protein on the cell surface. In a preferred embodiment the marker is a genetic or proteomic expression, which can be detected and correlated with the identity of the cell. The markers may be referred to by gene. This can readily be translated into the expression of the corresponding mRNA and proteins.

As used herein, the term "negative" or "-" when used in reference to any marker such as a surface protein or transcription factor disclosed herein refers to the marker not being expressed in a cell or a population of cells, while the term "weak" or "low" refers to the marker being expressed at a reduced level in a cell as compared to the mean expression of the marker in a population of cells or as compared to a reference sample.

As used herein, the term "double negative" refers to two markers not being expressed in a cell or a population of cells.

As used herein, the term "positive" or "+" when used in reference to any marker such as a surface protein or transcription factor disclosed herein refers to the marker being expressed in a cell or a population of cells, while the term "high" or "strong" refers to the marker being expressed at an increased level in a cell as compared to the mean expression of the marker in a population of cells or as compared to a reference sample.

As used herein, the term "double positive" refers to two markers being expressed in a cell or a population of cells.

### Method

As used herein, the term "method" refers to a process comprising or consisting of one or more or a series of steps performed to obtain the desired outcome or product. Throughout this application the terms "method" and "protocol", when referring to processes for differentiating cells, may be used interchangeably.

As used herein, the term "step" in relation to methods as described herein is to be understood as a stage, where something is undertaken and/or an action is performed. It will be understood by one of ordinary skill in the art when the step(s) to be performed is a first step or an intermediate step occurring between one or more steps or a final step and/or the steps undertaking are concurrent and/or successive and/or continuous.

As used herein, the term "day" and similarly day *in vitro* (DIV), in reference to the protocols, refers to a specific time for carrying out certain steps during the differentiation procedure. It will be understood by one of ordinary skill in the art when the day is expressed alternatively in hours.

In general, and unless otherwise stated, "day 0" refers to the initiation of the protocol, this be by for example but not limited to plating the cells or transferring the cells to an incubator or contacting the cells in their current cell culture medium with a compound prior to transfer of the cells. Typically, the initiation of the protocol will be by transferring the cells, such as e.g. undifferentiated stem cells, definitive endoderm cells, pancreatic endoderm cell, pancreatic endocrine progenitor (EP) cells or pancreatic endocrine (PEC) cells to a different cell culture medium and/or container such as, but not limited to, by plating or incubating, and/or with the first contacting of the cells with a compound or compounds that affects the undifferentiated stem cells in such a way that a differentiation process is initiated.

When referring to "day X", such as day 1, day 2 etc., it is relative to the initiation of the protocol at day 0. One of ordinary skill in the art will recognize that unless otherwise specified the exact time of the day for carrying out the step may vary. Accordingly, "day X" is meant to encompass a time span such as of +/-10 hours, +/-8 hours, +/-6 hours, +/-4 hours, +/-2 hours, or +/-1 hours. One of ordinary skill in the art will understand that the days can be used in combination with the cell stage for e.g. DE02 means definitive endoderm at day 2, DE04 means definitive endoderm at day 4 etc.

As used herein, the phrase "from at about day X to at about day Y" refers to a day at which an event starts from. The phrase provides an interval of days on which the event may start from. For example, if "cells are contacted with a differentiating factor from at about day 3 to at about day 5" then this is to be construed as encompassing all the options: "the cells are contacted with a differentiating factor from about day 3", "the cells are contacted with a differentiating factor from about day 4", and "the cells are contacted with a differentiating factor from about day 5". Accordingly, this phrase should not be construed as the event only occurring in the interval from day 3 to day 5. This applies *mutatis mutandis* to the phrase "to at about day X to at about day Y".

### Identifying or detecting

As used herein, the term "identifying" or "detecting" refers to establishing the presence of stromal-like cells using one or several conventional methods.

In one embodiment, detecting is done by immunofluorescence microscopy-based methods.

In another embodiment, detecting is done by way of identifying one or more markers.

In one embodiment, detecting is done by identifying one or more markers of the stromal like cells, the markers selected from a group consisting of COL1A1, COL1A2, COL3A1, COL6A1, COL6A2, FN1, THY1, VIM, NES, CDH2, PDGFRA, PDGFRB, MEIS1, SIX1, PAX2, PAX8, PRRX1, PCOLCE, VCAN, DDR1, DDR2, SPARC and CDH11.

In one embodiment, detecting is done by flow cytometry.

In another embodiment, detecting is done by gene expression analysis.

In one embodiment, detecting is done by measuring metabolites in cell culture medium.

In another embodiment, detecting is done by measuring cell numbers.

Candidate list of genes expressed by stromal like cells is shown in table 1 below.

**TABLE 1**

| Gene name | Ensembl Gen ID |
|---|---|
| COL1A1 | ENSG00000108821 |
| COL1A2 | ENSG00000164692 |
| COL3A1 | ENSG00000168542 |
| COL6A1 | ENSG00000142156 |
| COL6A2 | ENSG00000142173 |
| FN1 | ENSG00000115414 |
| THY1 | ENSG00000154096 |
| VIM | ENSG00000026025 |
| NES | ENSG00000132688 |
| CDH2 | ENSG00000170558 |
| PDGFRA | ENSG00000134853 |
| PDGFRB | ENSG00000113721 |
| MEIS1 | ENSG00000143995 |
| SIX1 | ENSG00000126778 |
| PAX2 | ENSG00000075891 |
| PAX8 | ENSG00000125618 |
| PRRX1 | ENSG00000116132 |
| PCOLCE | ENSG00000106333 |
| VCAN | ENSG00000038427 |
| DDR1 | ENSG00000204580 |
| DDR2 | ENSG00000162733 |
| SPARC | ENSG00000113140 |
| CDH11 | ENSG00000140937 |

In one embodiment, method for identifying contaminant stromal like cells in a population of non-stromal cells differentiated from human pluripotent stem cells comprising the steps of:
(i) selectively expanding the stromal like cells in the population of cells in a culture medium comprising MMCDB131, bFGF and FBS; and
(ii) detecting the contaminant stromal like cells.

In one embodiment, method for identifying contaminant stromal like cells in a population of PEC differentiated from human pluripotent stem cells comprising the steps of:
(i) selectively expanding the stromal like cells in the population of cells in a culture medium comprising MMCDB131, bFGF and FBS; and
(ii) detecting the contaminant stromal like cells.

In one embodiment, method for identifying contaminant stromal like cells in a population of PEC differentiated from human pluripotent stem cells comprising the steps of:
(i) selectively expanding the stromal like cells in the population of cells in a culture medium comprising MMCDB131, bFGF and FBS; and
(ii) detecting the contaminant stromal like cells by identifying one or more markers of the stromal like cells selected from a group consisting of COL1A1, COL1A2, COL3A1, COL6A1, COL6A2, FN1, THY1, VIM, NES, CDH2, PDGFRA, PDGFRB, MEIS1, SIX1, PAX2, PAX8, PRRX1, PCOLCE, VCAN, DDR1, DDR2, SPARC and CDH11.

In one embodiment, method for identifying contaminant stromal like cells in a population of PEC differentiated from human pluripotent stem cells comprising the steps of:
(i) selectively expanding the stromal like cells in the population of cells in a culture medium comprising MMCDB131, 2ng/ml to 200ng/ml bFGF and FBS; and
(ii) detecting the contaminant stromal like cells by identifying one or more markers of the stromal like cells selected from a group consisting of COL1A1, COL1A2, COL3A1, COL6A1, COL6A2, FN1, THY1, VIM, NES, CDH2, PDGFRA, PDGFRB, MEIS1, SIX1, PAX2, PAX8, PRRX1, PCOLCE, VCAN, DDR1, DDR2, SPARC and CDH11.

In one embodiment, method for identifying contaminant stromal like cells in a population of PEC differentiated from human pluripotent stem cells comprising the steps of:
(i) selectively expanding the stromal like cells in the population of cells in a culture medium comprising MMCDB131, 20ng/ml bFGF and FBS; and
(ii) detecting the contaminant stromal like cells by identifying one or more markers of the stromal like cells selected from a group consisting of COL1A1, COL1A2, COL3A1, COL6A1, COL6A2, FN1, THY1, VIM, NES, CDH2, PDGFRA, PDGFRB, MEIS1, SIX1, PAX2, PAX8, PRRX1, PCOLCE, VCAN, DDR1, DDR2, SPARC and CDH11.

In one embodiment, method for identifying contaminant stromal like cells in a population of cardiomyocytes differentiated from human pluripotent stem cells comprising the steps of:
(i) selectively expanding the stromal like cells in the population of cells in a culture medium comprising MMCDB131, bFGF and FBS; and
(ii) detecting the contaminant stromal like cells.

### Culturing stem cells

As used herein, the term "culturing" refers to a continuous procedure, which is employed throughout the method to maintain the viability of the cells at their various stages. After the cells of interest have been isolated from, for example but not limited to, living tissue or embryo, they are subsequently maintained under carefully controlled conditions. These conditions vary for each cell type, but generally consist of a suitable vessel with a substrate and/or medium that supplies the essential nutrients (amino acids, carbohydrates, vitamins, minerals), growth factors, hormones, and gases (CO₂, O₂), and regulates the physiochemical environment (pH buffer, osmotic pressure, temperature).

As used herein, the term "selectively expanding" or "selective expansion" or "selectively expanded" means expanding, growing or proliferating only specific cells in a cell population whereas the remaining cells do not expand, proliferate or grow.

In one embodiment, the cells that are selectively expanding are contaminant cells.

In one embodiment, the cells that are selectively expanding are stromal like cells.

In one embodiment, the cells that are selectively expanding is performed for at least 8 days.

In one embodiment, the step of selectively expanding is performed for 8 days.

In another embodiment, the step of selectively expanding is performed for at least 10 days.

In another embodiment, the step of selectively expanding is performed for 10 days.

In another embodiment, the step of selectively expanding is performed for at least 12 days.

In another embodiment, the step of selectively expanding is performed for 12 days.

In another embodiment, the step of selectively expanding is performed for at least 14 days.

In another embodiment, the step of selectively expanding is performed for 14 days.

In another embodiment, the step of selectively expanding is performed for 14 days.

As used herein, the term "culture medium" or "cell culture medium" refers to a liquid or gel designed to support the growth of cells. Cell culture media generally comprise an appropriate source of energy and compounds which regulate the cell cycle.

In one embodiment, the culture medium comprises a basal medium, selected from a group consisting of MCDB131, DMEM and F12.

In another embodiment, the culture medium comprises MCDB131 as basal medium.

As used herein, the term "supplement" refers to a culture medium additive that can improve mammalian cell growth and viability.

In one embodiment, a supplement is selected from a group consisting of Fetal Bovine Serum (FBS), ITS-X, and B27.

In one embodiment, culture medium comprises FBS as a supplement.

As used herein, the term "growth factors" refers to a compound added to a culture medium to stimulate and enhance survival or growth of cells in the culture medium.

Exemplary growth factors include Fibroblast Growth Factor (FGF) family including bovine Fibroblast Growth Factor (bFGF), Epidermal Growth Factor (EGF), Platelet-derived Growth Factor (PDGF), Sonic Hedgehog (SHH) and Sphingosine-1-phosphate.

In one embodiment, one or more growth factor(s) is added to a culture medium to stimulate and enhance the survival and growth of contaminant stromal like cells in the culture medium.

In one aspect the growth factor is a FGF family member.

In one aspect the growth factor is basic fibroblast growth factor (bFGF)

In another embodiment, bFGF is added to a culture medium to stimulate and enhance the survival and growth of contaminant stromal like cells in the culture medium.

In one embodiment, one or more growth factor(s) is added to a culture medium to stimulate and enhance the survival and growth of only contaminant stromal like cells, not the non-stromal cells in the culture medium.

In one embodiment, one or more supplements and growth factors are added to a culture medium to stimulate and enhance the survival and growth of contaminant stromal like cells in the culture medium.

In one embodiment, one or more supplements and growth factor(s) is added to a culture medium to stimulate and enhance the survival and growth of only contaminant stromal like cells, not the non-stromal cells in the culture medium.

In one embodiment, selectively expanding the stromal like cells in the population of cells in a culture medium comprising one or more growth factors and/or supplements.

In one embodiment, selectively expanding the stromal like cells in the population of cells in a culture medium comprising bFGF and FBS.

In one embodiment, selectively expanding the stromal like cells in the population of cells in a culture medium comprising 2ng/ml to 200ng/ml bFGF and FBS.

In one embodiment, selectively expanding the stromal like cells in the population of cells in a culture medium comprising 20ng/ml bFGF and FBS.

As used herein, the term "incubator" refers to any suitable incubator that may support a cell culture. Non-limiting examples include culture dish, petri dish and plate (microtiter plate, microplate, deep well plate etc. of 6 well, 24 well, 48 well, 96 well, 384 well, 9600 well and the like), flask, chamber slide, tube, Cell Factory, roller bottle, spinner flask, hollow fiber, microcarrier, or bead.

As used herein, the term "providing or obtaining stem cells" when referred to in a protocol means obtaining a batch of cells by methods such as described above and optionally transferring the cells into a different environment such as by seeding onto a new substrate. One of ordinary skill in the art will readily recognize that stem cells are fragile to such transfer and the procedure requires diligence and that maintaining the stem cells in the origin cell culture medium may facilitate a more sustainable transfer of the cells before replacing a cell culture medium with another cell culture medium more suitable for a further differentiation process.

As used herein, the term *"in vitro"* means that the cells are provided and maintained outside of the human or animal body, such as in a vessel like a flask, multiwell or petri dish. It follows that the cells are cultured in a cell culturing medium.

### Differentiating stem cells

As used herein, the term "differentiation" or "differentiate" or "differentiating" refers broadly to the process wherein cells progress from an undifferentiated state or a state different from the intended differentiated state to a specific differentiated state, e.g. from an immature state to a less immature state or from an immature state to a mature state, which may occur continuously as the method is performed. Changes in cell interaction and maturation occur as cells lose markers of undifferentiated cells or gain markers of differentiated cells. Loss or gain of a single marker can indicate that a cell has "fully differentiated". "Fully differentiated" cells are the final stage of a developmental lineage and cannot further differentiate.

The term "differentiation factor" refers to a compound added to stem cells to enhance their differentiation into mature cells. In one embodiment, differentiation factor is added to pluripotent stem cell to enhance their differentiation into definitive endoderm cell.

Exemplary differentiation factors include hepatocyte growth factor, keratinocyte growth factor, exendin-4, basic fibroblast growth factor, insulin-like growth factor-1, epidermal growth factor platelet-derived growth factor, and glucagon-like peptide 1. In aspects of the invention, differentiation of the cells comprises culturing the cells in a medium comprising one or more differentiation factors.

In one embodiment, differentiation of the cells comprises culturing the cells in a medium comprising one or more differentiation factors.

As used herein, the term "for further differentiation" or "capable of further differentiation" refers to the ability of a differentiated cell to progress from a differentiated state to a more mature state either directly or in a stepwise manner e.g. definitive endoderm "for further differentiation" into insulin producing cells means that the definitive endoderm cells are capable of differentiating into insulin producing cells when subjected to a suitable differentiation protocol.

As used herein, by the term "contacting" in reference to culturing or differentiating cells is meant exposing the cells to e.g. a specific compound by placing the specific compound in a location that will allow it to touch the cell in order to produce "contacted" cells. The contacting may be accomplished using any suitable means. A non-limiting example of contacting is by adding the compound to a cell culture medium of the cells. The contacting of the cells is assumed to occur as long as the cells and specific compound are in proximity, e.g. the compound is present in a suitable concentration in the cell culture medium.

### Known protocols for the individual differentiation steps from hPSC to pancreatic endocrine cells

In the following, references are given to already known protocols for the individual differentiation steps from hPSC to pancreatic endocrine cells. hPSC are differentiated towards pancreatic endocrine (PEC) cells in a stepwise manner through distinct stages. These stages include definitive endoderm (DE), pancreatic endoderm (PE), endocrine progenitor (EP) cells (EP) and finally to pancreatic islet cells (also denoted PEC) *(*Madsen et al. - Nat Biotechnol. - 2006 Dec, 24(12):1481-3)*.* DE is commonly derived by treating hPSC with transforming growth factor β and WNT/β-Catenin agonists (D'Amour et al. - Nat Biotechnol. - 2005 Dec; 23(12):1534-41*,* Rezania et al. - Diabetes - 20 2011 Jan;60(1):239-47*,* Kubo et al. - Development - 2004 Apr;131(7):1651-62*,* Rezania et al. - Nat Biotechnol. - 2014 Nov;32(11):1121-33 Funa et al. - Cell Stem Cell. - 2015 Jun 4;16(6):639-52*).* The protocol described in WO2012/175633 is incorporated herein by reference in its entirety. DE is further specified into PDX1+ NKX6.1+ PE population in vitro.

Fibroblast growth factor, retinoic acid, sonic hedgehog, epidermal growth factor and bone morphogenic protein signalling pathways have all been implicated in pancreas development and manipulation of these pathways at distinct stages of the differentiation promote highly enriched populations of PE (D'Amour et al. - Nat Biotechnol. - 2006 Nov;24(11):1392-401*,* Kroon et al. - Nat Biotechnol. - 2008 Apr;26(4):443- 52*,* Nostro et al. - Development - 2011 Mar;138(5):861-71*,* Rezania et al. - Diabetes - 2012 Aug;61(8):2016-29*,* Mfopou et al. - Gastroenterology - 2010 Jun;138(7):2233-45*,* Ameri et al. - Stam cells - 2010 Jan;28(1):45-56*,* Russ et al. - EMBO J. - 2015 Jul 2;34(13):1759-72*).* PE inducing protocols is described in WO2014/033322, which is incorporated herein by reference in its entirety. Additional pathways including protein kinase C, Nicotinamide, WNT, Rho associated kinase and TGFβ have also been identified to participate in the specification of hPSC towards the pancreatic lineage (Chen et al.- Nat Chem Biol. - 2009 Apr;5(4):258-65*,* Rezania et al.-Diabetes - 2012 Aug;61(8):2016-29*,* Rezania et al. - Stem Cell - 2013 Nov;31(11):2432-42*,* Nostro et al. - Stem Cell Reports - 2015 Apr 14;4(4):591-604*,* Sharon et al. - Cell Rep. - 2019 May 21;27(8):2281-2291.e5, 35 Toyoda et al. - Stem Cell Rep. - 2017 Aug 8;9(2):419-428*).* Pancreatic endocrine specification from PE is dependent on the expression of the transcription factor NEUROG3 *(*McGrath et al. - Diabetes - 2015 Jul;64(7):2497-505*,* Zhang et al. - Dev. Cell - 2019 Aug 5;50(3):367-380.e7*.).*

Several approaches have been explored to induce EP as well as PEC from PE. Culturing PE on air-liquid interface culture resulted in upregulation of NEUROG3 5 transcript, as well as the pancreatic hormones insulin (INS) and glucagon (GCG), compared with cells cultured in planar culture Rezania et al. - Nat Biotechnol. - 2014 Nov;32(11):1121-33*).* Expression of a NEUROG3 transgene in PE was shown to induce endocrine differentiation *(*Zhu et al. - Cell Stem Cell. - 2016 Jun 2;18(6):755-768*).* Modulation of the actin cytoskeleton as well as dispersion of PE to single cells followed by reaggregation cells to clusters can induce NEUROG3 10 expression and differentiation to EP and hPSC-endocrine cells *(*Mamidi - Nature. - 2018 Dec;564(7734):114-118*,* Hogrebe et al. - Nat Biotechnol. - 2020 Apr;38(4):460-470*).* Inhibition of TGFβ signalling and Notch signalling progressed PE to a pancreatic endocrine phenotype (Rezania et al. - Diabetes. - 2011 Jan;60(1):239-47*,* Nostro et al. - Development. - 2011 Mar;138(5):861-71*,* Pagliuca et al. - Cell. - 2014 Oct 9;159(2):428-39*,* Rezania et al. - Nat 15 Biotechnol. - 2014 Nov;32(11):1121-33*,* Rezania et al. - Differentiation of human embryonic stem cells - 2015 Jun 23)*.* However, permitting TGFβ signalling appears to be required for differentiation to more mature beta-like cells (*Velazco-*Cruz et al. - Stem Cell Reports. - 2019 Feb 12;12(2):351- 365)*.* Several other signalling pathways has been shown to promote EP induction and further differentiation to hPSC-endocrine cells. Bromodomain and extraterminal (BET) protein inhibition with I-BET151 or JQ1 enhanced the number of NEUROG3 endocrine progenitor (EP) cells (Huijbregts et al. - Diabetes. - 2019 Apr;68(4):761-773*).* Sodium cromoglicate (SCG), was identified in a small molecule screen and induced pancreatic endocrine differentiation in PE through the inhibition of bone morphogenetic protein 4 signalling pathway *(*Kondo et al. - Diabetologia. - 2017 25 Aug;60(8):1454-1466*.). Bone morphogenetic protein has been implicated in endocrine induction (*Nostro et al. - Development. - 2011 Mar;138(5):861-71 Sharon et al. - Cell Rep. - 2019 May 21;27(8):2281-2291.e5*.)* and inhibitors of this pathway is commonly used in differentiation of hPSC to EP and PEC. Treatment of PE with the WNT-tankyrase inhibitor IWR1-endo increased the expression of endocrine markers and downregulated progenitor markers demonstrating that small_30 molecule WNT inhibitors increases the endocrine induction *(*Sharon et al. - Cell Rep. - 2019 May 21; 27(8): 2281-2291.e5*.).* Finally, specific pancreatic endocrine cell types resembling their in vivo counterparts have been derived and characterized in detail. A protocol for generating pancreatic endocrine progenitor cells is described in WO2015/028614 which is incorporated herein by reference in its entirety.

Glucagon expressing alpha-like cells derived from hPSC display molecular and functional characteristics of bona fide pancreatic alpha cells *(*Rezania et al. - 35 Diabetes - 2011 Jan;60(1):239-47*,* Peterson et al. - Nat Common. - 2020 May 7;11(1):2241*).* Differentiation protocols for maturing hPSC-derived beta-like cells that are capable of secreting insulin in response to elevated glucose concentrations have recently been reported *(*Rezania et al. - Nat Biotechnol. - 2014 Nov;32(11):1121-33*,* Pagliuca et al. - Cell. - 2014 Oct 9;159(2):428-39*,* Velazco-Cruz et al. - Stem Cell Reports. - 2019 Feb 12;12(2):351-365*,* Hogrebe et al. - Nat Biotechnol. - 2020 Apr;38(4):460-470*,* Liu et al. - Nat Commun. - 2021 Jun 7;12(1):3330*,* Nair et al. 5 - Nat Cell Biol. - 2019 Feb;21(2):263-274*).* Single cell gene expression analysis has delineated the differentiation path of hPSC towards PEC cells including beta-like cells *(*Petersen et al. - Stem Cell Reports. - 2017 Oct 10;9(4):1246-1261*,* Ramond et al. - Development. - 2018 Aug 15;145(16): dev165480*,* Docherty et al. - Diabetes. - 2021 Nov;70(11):2554-2567*,* Veres et al. - Nature. - 2019 10 May;569(7756):368-373*)* and detailed characterization of the hPSC-derived beta-like cells both in vitro and in vivo have revealed many similarities to bona fide pancreatic beta cells *(*Velazco-Cruz et al. - Stem Cell Reports. - 2019 Feb 12;12(2):351-365*,* Augsornworawat et al. - Cell Rep. - 2020 Aug 25;32(8):108067*,* Balboa et al. - Functional, metabolic and transcriptional maturation of stem cell derived beta cells - 2021.03.31.437748v1*).* Interestingly, formation of non-endocrine cells as well as non-pancreas enterochromaffin cells has recently been reported for protocols aiming and differentiation hPSC towards the pancreatic endocrine lineage *(*Petersen et al. - Stem Cell Reports. - 2017 Oct 10;9(4):1246-1261*,* Veres et al. - Nature. - 2019 May;569(7756):368-373*).*

The PEC may be further treated with a cryopreservation medium and lowering temperature to obtain cryopreserved single cells. A suitable method for cryopreservation of PEC is described in WO 2019/048690 to which reference is made and which is incorporated by reference in its entirety.

### Stem cell-derived products

As used herein, the term "differentiated cells" refers to cells such as pluripotent stem cells which have progressed from an undifferentiated state to a less immature state. Differentiated cells may be e.g., less immature specialized cell such as progenitor cells or matured fully into a specialized/terminal cell type.

As used herein, the term "cell lineage" refers to the developmental origin of a cell type or cell types starting from pluripotent stem cells and progressing to less immature cells and further to specialized/terminally differentiated cell types.

As used herein, the term "cell population" refers to a plurality of cells in the same culture. The cell population may be e.g., a mixture of cells of different types, or cells at various developmental stages such as cells at various maturity stages towards the same or similar specialized feature or it may be a more homogeneous composition of cells with common markers.

The invention is further described by the following non-limiting embodiments:
1. A method for identifying contaminant stromal like cells in a population of non-stromal cells differentiated from human pluripotent stem cells comprising the steps of:
   (i) selectively expanding the stromal like cells in the population of cells in a culture medium comprising one or more growth factors and/or supplements, and
   (ii) detecting the contaminant stromal like cells.
2. The method according to embodiment 1, wherein the stromal like cells are CHGA/cTnT⁻ /NES⁺ cells.
3. The method according to embodiment 1, wherein the contaminant stromal like cells are selected from a group consisting of fibroblasts, pericytes, mesenchymal stromal cells, adipocyte stromal cells, endothelial cells and smooth muscle cells.
4. The method according to embodiment 1, wherein the contaminant stromal like cells are fibroblasts.
5. The method according to embodiment 1, wherein the contaminant stromal like cells are pericytes.
6. The method according to embodiment 1, wherein the contaminant stromal like cells are mesenchymal stromal cells.
7. The method according to embodiment 1, wherein the contaminant stromal like cells are adipocyte stromal cells.
8. The method according to embodiment 1, wherein the contaminant stromal like cells are endothelial cells.
9. The method according to embodiment 1, wherein the contaminant stromal like cells are smooth muscle cells.
10. The method according to embodiment 1, wherein the population of non-stromal cells differentiated from human pluripotent stem cells is selected from a group consisting of pancreatic endocrine cells, cardiomyocytes, dopaminergic progenitors and retinal pigmented epithelial cells.
11. The method according to embodiment 1, wherein the population of non-stromal cells differentiated from human pluripotent stem cells is pancreatic endocrine cells.
12. The method according to embodiment 1, wherein the population of non-stromal cells differentiated from human pluripotent stem cells is cardiomyocytes.
13. The method according to embodiment 1, wherein the population of non-stromal cells differentiated from human pluripotent stem cells is dopaminergic progenitors.
14. The method according to embodiment 1, wherein the population of non-stromal cells differentiated from human pluripotent stem cells is retinal pigmented epithelial cells.
15. The method according to embodiment 1, wherein one or more growth factors are selected from a group consisting of bFGF, EGF, PDGF, SHH and Sphingosine-1-phosphate.
16. The method according to embodiment 15, wherein the growth factors bFGF, EGF, PDGF, SHH are present in a concentration range of from 2ng/ml to 200ng/ml.
17. The method according to embodiment 16, wherein bFGF is present in a concentration range of from 2ng/ml to 200ng/ml.
18. The method according to embodiment 17, wherein bFGF is present in a concentration of 20ng/ml.
19. The method according to embodiment 16, wherein EGF is present in a concentration of about 50ng/ml.
20. The method according to embodiment 16, wherein EGF is present in a concentration of 50ng/ml.
21. The method according to embodiment 16, wherein PDGF is present in a concentration of about 200ng/ml.
22. The method according to embodiment 16, wherein PDGF is present in a concentration of 200ng/ml.
23. The method according to embodiment 16, wherein SHH is present in a concentration of about 50ng/ml.
24. The method according to embodiment 16, wherein SHH is present in a concentration of 50ng/ml.
25. The method according to embodiment 15, wherein Sphingosine-1-phosphate is present in a concentration of about 1uM.
26. The method according to embodiment 15, wherein Sphingosine-1-phosphate is present in a concentration of 1uM.
27. The method according to embodiment 1, wherein the culture medium comprises a basal medium selected from a group consisting of MCDB131, DMEM and F12.
28. The method according to embodiment 27, wherein the culture medium comprises basal medium MCDB131.
29. The method according to embodiment 1, wherein the culture medium comprises a matrix selected from a group consisting of fibronectin, iMatrix and collagen.
30. The method according to embodiment 1, wherein the culture medium comprises a supplement selected from a group consisting of fetal bovine serum, ITS-X and B27.
31. The method according to embodiment 30, wherein the culture medium comprises a supplement selected from a group consisting of fetal bovine serum.
32. The method according to embodiment 1, wherein the culture medium comprises a basal medium, one or more growth factors and supplements.
33. The method according to embodiment 1, wherein the culture medium comprises MCDB131, bFGF and fetal bovine serum.
34. The method according to embodiment 1, wherein the step (i) of selectively expanding is performed for at least 8 days.
35. The method according to embodiment 1, wherein the step (i) of selectively expanding is performed for 8 days.
36. The method according to embodiment 1, wherein the step (i) of selectively expanding is performed for at least 10 days.
37. The method according to embodiment 1, wherein the step (i) of selectively expanding is performed for 10 days.
38. The method according to embodiment 1, wherein the step (i) of selectively expanding is performed for at least 12 days.
39. The method according to embodiment 1, wherein the step (i) of selectively expanding is performed for 12 days.
40. The method according to embodiment 1, wherein the step (i) of selectively expanding is performed for at least 14 days.
41. The method according to embodiment 1, wherein the step (i) of selectively expanding is performed for 14 days.
42. The method according to embodiment 1, wherein the step (ii) of detecting the contaminant stromal like cells is by way of immunofluorescence microscopy-based methods.
43. The method according to embodiment 1, wherein the step (ii) of detecting the contaminant stromal like cells is by identifying one or more markers of stromal cells.
44. The method according to embodiment 1, wherein detecting the stromal like cells is by identifying one or more markers of the stromal like cells selected from a group consisting of COL1A1, COL1A2, COL3A1, COL6A1, COL6A2, FN1, THY1, VIM, NES, CDH2, PDGFRA, PDGFRB, MEIS1, SIX1, PAX2, PAX8, PRRX1, PCOLCE, VCAN, DDR1, DDR2, SPARC and CDH11.
45. The method according to any one of the preceding embodiments comprising a further step (iii) of purifying the population of homogenous non-stromal cells differentiated from human pluripotent stem cells by removing the contaminant stromal like cells.
46. A culture medium for selectively increasing the proportion of stromal like cells in a population of non-stromal cells differentiated from human pluripotent stem cells.
47. The culture medium according to embodiment 46, comprising one or more growth factors and/or supplements.
48. The culture medium according to embodiment 46, wherein one or more growth factors are selected from a group consisting of bFGF, EGF, PDGF, SHH and Sphingosine-1-phosphate.
49. The culture medium according to embodiment 48, wherein the growth factors bFGF, EGF, PDGF, SHH are present in a concentration range of from 2ng/ml to 200ng/ml.
50. The culture medium according to embodiment 49, wherein bFGF is present in a concentration range of from 2ng/ml to 200ng/ml.
51. The culture medium according to embodiment 50, wherein bFGF is present in a concentration of 20ng/ml.
52. The culture medium according to embodiment 49, wherein EGF is present in a concentration of about 50ng/ml.
53. The culture medium according to embodiment 49, wherein EGF is present in a concentration of 50ng/ml.
54. The culture medium according to embodiment 49, wherein PDGF is present in a concentration of about 200ng/ml.
55. The culture medium according to embodiment 49, wherein PDGF is present in a concentration of 200ng/ml.
56. The culture medium according to embodiment 49, wherein SHH is present in a concentration of about 50ng/ml.
57. The culture medium according to embodiment 49, wherein SHH is present in a concentration of 50ng/ml.
58. The culture medium according to embodiment 48, wherein Sphingosine-1-phosphate is present in a concentration of about 1uM.
59. The culture medium according to embodiment 48, wherein Sphingosine-1-phosphate is present in a concentration of 1uM.
60. The culture medium according to embodiment 46, wherein the culture medium comprises a basal medium selected from a group consisting of MCDB131, DMEM and F12.
61. The culture medium according to embodiment 60, wherein the culture medium comprises basal medium MCDB131.
62. The culture medium according to embodiment 46, wherein the culture medium comprises a matrix selected from a group consisting of fibronectin, iMatrix and collagen.
63. The culture medium according to embodiment 46, wherein the culture medium comprises a supplement selected from a group consisting of fetal bovine serum, ITS-X and B27.
64. The culture medium according to embodiment 63, wherein the culture medium comprises a supplement selected from a group consisting of fetal bovine serum.
65. The culture medium according to embodiment 46, wherein the culture medium comprises growth factor and supplement.
66. The culture medium according to embodiment 65, wherein the culture medium comprises bFGF and fetal bovine serum.
67. The culture medium according to embodiment 46, wherein the culture medium comprises a basal medium, one or more growth factors and supplements.
68. The culture medium according to embodiment 65, wherein the culture medium comprises MCDB131, bFGF and fetal bovine serum.
69. A cell population comprising non-stromal cells differentiated from human pluripotent stem cells and less than 1% of contaminant stromal like cells for use as a medicament.
70. The cell population according to embodiment 69, comprising non-stromal cells differentiated from human pluripotent stem cells and less than 0.5% of contaminant stromal like cells for use as a medicament.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

### Examples

The following are non-limiting examples for carrying out the invention.

### Example 1: Differentiation of human pluripotent stem cells

Human pluripotent stem cells (hPSC) can be differentiated to cell types of the three germ layers. Protocols for differentiation of hPSC to specific cell types have been published, including cardiomyocytes *(*Continuous WNT Control Enables Advanced hPSC Cardiac Processing and Prognostic Surface Marker Identification in Chemically Defined Suspension Culture, Stem Cell Reports, 2019 Oct 8; 13(4):775*.),* pancreatic endocrine cells *(*Reversal of diabetes with insulin-producing cells derived in vitro from human pluripotent stem cells, Nature Biotechnology, 32, pages 1121-1133 (2014*);* Generation of functional human pancreatic β cells in vitro, Cell. 2014 Oct 9; 159(2): 428-439; Charting cellular identity during human in vitro β-cell differentiation; Nature. 2019 May; 569(7756): 368-373*)* and dopaminergic progenitors *(*Preclinical quality, safety, and efficacy of a human embryonic stem cell-derived product for the treatment of Parkinson's disease, STEM-PD, Cell Stem Cell Volume 30, Issue 10, 5 October 2023, Pages 1299-1314*;* Preclinical Efficacy and Safety of a Human Embryonic Stem Cell-Derived Midbrain Dopamine Progenitor Product, MSK-DA01, Cell Stem Cell Volume 28, Issue 2, 4 February 2021, Pages 217-229*;* Preclinical and dose-ranging assessment of hESC-derived dopaminergic progenitors for a clinical trial on Parkinson's disease, Cell Stem Cell, Volume 31, Issue 1, 4 January 2024, Pages 25-38*;* Preclinical study of induced pluripotent stem cell-derived dopaminergic progenitor cells for Parkinson's disease, Nat Common. 2020; 11: 3369). Following differentiation, hPSC-derived cell types (cells) are dissociated into a single cell suspension using enzymatic digestion. Briefly, cells are washed with phosphate-buffered saline without calcium and magnesium (PBS-/-, Gibco, product no. 14190-169). PBS-/- is removed and an enzymatic digestion agent is added to the cells and incubated for 37°C until cells are dispersed to single cells. Examples of enzymatic digestion agent are TrypLE Select (Gibco, product no. 12563-011) or Accutase Cell detachment solution (STEMCELL Technologies, product no. 07920). The enzymatic digestion is quenched by addition of excess culture medium and cells are pelleted by centrifugation. Cell number and viability is determined using an automated cell counter.

### Example 2: Stromal like cell assay

hPSC-derived cell types (cells) in single cell suspension from example 1 are washed and subsequently resuspended in stromal like cell culture medium. In certain examples stromal like cell culture medium contains MCDB131 medium (Gibco, product no. 10372-019) supplemented with 1% (v/v) Glutamax (Gibco, cat. no. 35050038), 0.1% (v/v) penicillin-Streptomycin (P/S Gibco, product no. 15140-122) and 2% (v/v) fetal bovine serum (Sigma, product no. F2442). 20ng/ml of human recombinant basic fibroblast growth factor (bFGF, Peprotech, product no. 100-18B) is added to the medium. iMatrix-511MG (Nippi, product. no. 890-005) is added to the medium used for seeding cells, but not for subsequent medium change. Cells are seeded onto standard tissue culture plastic vessels. Seeding concentration can be varied across a wide range, but cells are often seeded at 10.000 cells/cm². After seeding, cells are incubated at 37°C, 5% CO₂. Medium is replenished every other day. Emergence and expansion of stromal like cells is monitored regularly using bright-field microscopy. In certain examples cells are cultured for a defined number of days to allow for sufficient expansion of stromal like cells for subsequent detection. An example of culture time is 10 days, after where the cell culture is harvested for detection of stromal like cells. In certain examples the cell culture can be passaged, and stromal like cell can be further expanded as shown in fig. 3 and 4. Briefly, medium is aspirated from cell cultures and cells are subsequently washed with PBS-/-. PBS-/- is removed and an enzymatic digestion agent is added to the cells and incubated for 37°C until cells are dispersed to single cells. The digestion is terminated by adding excess stromal medium (MCDB131 + Glutamax + P/S + FBS), cells are transferred to a centrifugation tube and pelleted. Cells are resuspended in stromal medium and cell number and viability is determined using an automated cell counter. Cells are seeded according to description above. Stromal like cells can be expanded across multiple passages and cryopreserved.

### Example 3: Detection of stromal like cells

Stromal like cells can be detected using a variety of methods, including flow cytometry, immunocytochemistry, and gene expression analysis. In one example, cell cultures are fixed directly on the tissue culture plastic using formaldehyde or other fixative agents. Cells are subsequently permeabilized and stained with antibodies directed against stromal like cell specific antigens. One of the several markers for stromal like cells is Nestin *(NES).* One of the markers for endocrine cells is FOXA2 *(FOXA2).* Secondary antibodies coupled to fluorescence molecules allows for the detection of cells expressing the specific antigens. Nuclei dyes such as DAPI or Hoechst 33342 allows for detection of all cells in the culture. The number of stromal like cells can be quantified using cell imaging reader platforms such as BioTek Cutation C10.

Expansion of stromal like cells can also be detected using various methods of cell growth. Such methods include determining the number of cells present in a culture after a defined number of culture days or measuring consumption of nutrients (e.g. glucose) and accumulation of metabolites (e.g. lactate) in culture medium. Alternatively, detection of stromal like cells can be carried out by any suitable detection method known in the art.

### Example 4: Combinatorial medium screen

To identify optimal culture conditions for stromal like cells, combinations of basal medium, medium supplements, extracellular matrix and growth factors were evaluated by assessing stromal like cell expansion over four days. Expanded stromal like cells were seeded at 500 cells/well in 96-well plates precoated with specific extracellular matrixes. Cells were seeded in MCDB131 medium containing 1% Glutamax, 2% FBS and 0.1% P/S and allowed to adhere to the tissue culture plastic for 2h at 37°C, 5% CO₂. Medium was then aspirated and replaced with different combinations of basal medium, medium supplements and growth factors listed in table 2 below.

**TABLE 2**

| Conditio n | Medium + supplement | | | Matrix | | Signaling pathway | |
|---|---|---|---|---|---|---|---|
| | Basal mediu m | Suppleme nt | Concentratio n | Matrix | Concentr ation | Growth factor | Final concentr ation |
| 1. | MCDB1 31 | FBS | 2% | Fibronecti n | 5ug/cm2 | bFGF | 20ng/ml |
| 2. | DMEM/ F12 | FBS | 2% | iMatrix | 5ug/cm2 | SHH | 50ng/ml |
| 3. | MCDB1 31 | ITS-X | 1% | Collagen | 5ug/cm2 | Sphing osine-1-phosph ate | 1uM |
| 4. | DMEM( HG) | B27 | 1% | iMatrix | 5ug/cm2 | 5ug/cm 2 | 20ng/ml |
| 5. | MCDB1 31 | B27 | 1% | Collagen | 5ug/cm2 | SHH | 50ng/ml |
| 6. | DMEM/ F12 | ITS-X | 1% | iMatrix | 5ug/cm2 | PDGF-AA | 200ng/ml |
| 7. | DMEM/ F12 | B27 | 1% | Fibronecti n | 5ug/cm2 | EGF | 50ng/ml |
| 8. | MCDB1 31 | ITS-X | 1% | Fibronecti n | 5ug/cm2 | SHH | 50ng/ml |
| 9. | MCDB1 31 | B27 | 1% | Fibronecti n | 5ug/cm2 | PDGF-AA | 200ng/ml |
| 10. | MCDB1 31 | Empty | - | iMatrix | 5ug/cm2 | EGF | 50ng/ml |
| 11. | DMEM/ F12 | ITS-X | 1% | Fibronecti n | 5ug/cm2 | CHIR | 2uM |
| 12. | DMEM( HG) | ITS-X | 1% | iMatrix | 5ug/cm2 | EGF | 50ng/ml |
| 13. | DMEM( HG) | Empty | - | Fibronecti n | 5ug/cm2 | Sphing osine-1-phosph ate | 1uM |
| 14. | MCDB1 31 | FBS | 2% | Collagen | 5ug/cm2 | EGF | 50ng/ml |
| 15. | DMEM/ F12 | B27 | 1% | Collagen | 5ug/cm2 | CHIR | 2uM |
| 16. | DMEM( HG) | Empty | - | Collagen | 5ug/cm2 | SHH | 50ng/ml |
| 17. | DMEM( HG) | FBS | 2% | Fibronecti n | 5ug/cm2 | CHIR | 2uM |
| 18. | DMEM( HG) | B27 | 1% | iMatrix | 5ug/cm2 | Sphing osine-1-phosph ate | 1uM |
| 19. | DMEM/ F12 | FBS | 2% | iMatrix | 5ug/cm2 | Sphing osine-1-phosph ate | 1uM |
| 20. | DMEM( HG) | FBS | 2% | Collagen | 5ug/cm2 | PDGF-AA | 200ng/ml |
| 21. | DMEM/ F12 | Empty | - | Collagen | 5ug/cm2 | PDGF-AA | 200ng/ml |
| 22. | DMEM/ F12 | Empty | - | Fibronecti n | 5ug/cm2 | bFGF | 20ng/ml |
| 23. | DMEM( HG) | ITS-X | 1% | Collagen | 5ug/cm2 | bFGF | 20ng/ml |
| 24. | MCDB1 31 | Empty | - | iMatrix | 5ug/cm2 | CHIR | 2uM |

Cells were cultured for 2 days at 37°C, 5% CO₂ and medium was replenished with fresh medium according to table 2. After four days of culture the total number of cells were quantified using the CellTiter-Glo Luminescent Cell Viability Assay (Promega, cat. no. G7570) as shown in Fig. 9. Briefly, half of the medium was aspirated from the wells and a similar volume of CellTiter-Glo Luminescent reagent was added to the wells. Plates were mixed for two minutes on an orbital shaker and subsequently incubated for 10min at RT before recording the luminescence signal using a microplate reader. Baseline was established by recording the luminescence signal for stromal like cells after the initial 2h seeding step. Expansion of cells had occurred if the luminescence signal was higher at day four compared to the signal after 2h. The higher the luminescence signal, the more expansion had occurred.

### Example 5: Sensitivity of stromal like cell assay

To illustrate the higher sensitivity of the stromal like cell assay in detecting stromal like cells compared to conventional detection methods and to be able to predict the level of contaminating stromal like cells *in vivo,* a comparison between flow cytometry and stromal like cell assay was conducted. Three pancreatic endocrine cell samples (sample A, B and C) were evaluated by flow cytometry, using a marker for endocrine cells (CHGA) and a marker for stromal like cells (VIM). A very low number of VIM+/CHGA- stromal like cells were detected in the three samples (Fig.6A) making it difficult to determine the level of contaminating stromal like cells in the three samples. The same three samples were also evaluated using the stromal like cell assay (Fig.6B). Cells were cultured in 6-well tissue culture plates (6 wells pr. sample) for 10 days and subsequently stained for a marker for endocrine cells (FOXA2) and a marker for stromal like cells (NESTIN). The number of stromal cells were quantified using an BioTek Cytation C10 cell imaging reader. The number of stromal like cells quantified in the stromal like cell assay demonstrated that sample A had the highest proportion of stromal like cells, followed by sample B, whereas very few stromal like cells were detected in sample C (Fig. 6C). An *in vitro* assay for stromal like cells should ideally predict the levels of contaminating stromal like cells expanding *in vivo.* Thus, to evaluate the *in vivo* outcome, the three samples were also transplanted under the kidney capsule of immunocompromised mice for 12 weeks. The transplanted kidneys were explanted, and the cell composition of the grafts were evaluated using histology. Fig. 6D shows the quantified amount of contaminating stromal cells (stromal VIM/total human cells) in mice transplanted with the three samples. Mice receiving sample A had the highest amount of contaminating stromal like cell, followed by sample B, whereas mice receiving sample C had the lowest amount of contaminating stromal like cells in the grafts. Thus, the *in vivo* outcome is in alignment with the prediction of the stromal like cell assay, but not flow cytometry, demonstrating the superiority of the stromal like cell assay to a conventional detection method such as flow cytometry.

## Claims

1. A method for identifying contaminant stromal like cells in a population of non-stromal cells differentiated from human pluripotent stem cells comprising the steps of:
(i) selectively expanding the stromal like cells in the population of cells in a culture medium comprising one or more growth factors and/or supplements, and
(ii) detecting the contaminant stromal like cells.

2. The method according to claim 1, wherein the stromal like cells are CHGA⁻/cTnT⁻/NES⁺ cells.

3. The method according to claim 1, wherein the stromal like cells are selected from a group consisting of fibroblasts, pericytes, mesenchymal stromal cells, adipocyte stromal cells, endothelial cells, and smooth muscle cells.

4. The method according to claim 1, wherein the population of non-stromal cells differentiated from human pluripotent stem cells is selected from a group consisting of pancreatic endocrine cells, cardiomyocytes, dopaminergic progenitors and retinal pigmented epithelial cells.

5. The method according to claim 1, wherein one or more growth factors are selected from a group consisting of basic fibroblast growth factor, epidermal growth factor, platelet-derived growth factor, sonic hedgehog and Sphingosine-1-phosphate.

6. The method according to claim 5, wherein bGFG present in a concentration range of from 2ng/ml to 200ng/ml.

7. The method according to claim 1, wherein the step (i) of selectively culturing or expanding is performed for at least 8 days.

8. The method according to claim 1, wherein the culture medium comprises a basal medium selected from a group consisting of MCDB131, DMEM and F12.

9. The method according to claim 1, wherein the culture medium comprises a supplement selected from a group consisting of fetal bovine serum, ITS-X, and B27

10. The method according to claim 1, wherein the culture medium comprises a matrix selected from a group consisting of fibronectin, iMatrix and collagen.

11. The method according to claim 1, wherein the step (ii) of detecting the contaminant stromal like cells is by immunofluorescence microscopy-based methods.

12. The method according to claim 1, wherein the step (ii) of detecting the stromal like cells is by identifying one or more markers of the stromal like cells selected from a group consisting of COL1A1, COL 1A2, COL3A1, COL6A1, COL6A2, FN1, THY1, VIM, NES, CDH2, PDGFRA, PDGFRB, MEIS1, SIX1, PAX2, PAX8, PRRX1, PCOLCE, VCAN, DDR1, DDR2, SPARC and CDH11.

13. The method according to claim 1, further comprising a step (iii) of purifying the population of non-stromal cells differentiated from human pluripotent stem cells by removing the contaminant stromal like cells.

14. A culture medium for selectively increasing the proportion of stromal like cells in a population of cells comprising non-stromal cells differentiated from human pluripotent stem cells.

15. A cell population comprising non-stromal cells differentiated from human pluripotent stem cells and contaminant stromal like cells, wherein less than 1% of the cells are contaminant stromal like cells, for use as a medicament.
